(19) Europäisches Patentamt
European Patent Office
Office européen des brevets

(11) Veröffentlichungsnummer : **0 086 258**
**B1**

(12) # EUROPÄISCHE PATENTSCHRIFT

(45) Veröffentlichungstag der Patentschrift : **26.09.84**

(51) Int. Cl.³ : **C 07 C149/247**, C 07 B 19/00// C07D277/06

(21) Anmeldenummer : **82109858.9**

(22) Anmeldetag : **26.10.82**

(54) **Verfahren zur Trennung des Racemats (R,S)-Cystein.**

(30) Priorität : **26.01.82 DE 3202295**

(43) Veröffentlichungstag der Anmeldung :
**24.08.83 Patentblatt 83/34**

(45) Bekanntmachung des Hinweises auf die Patenterteilung : **26.09.84 Patentblatt 84/39**

(84) Benannte Vertragsstaaten :
**BE CH DE FR GB LI NL SE**

(56) Entgegenhaltungen :
**CH-A- 507 004**
**FR-A- 2 105 889**
**ANGEWANDTE CHEMIE, Band 93, Heft 8, 1981 Verlag Chemie, Weinheim J. MARTENS et al. "Einfache Synthese von racemischem Cystein"**
**Chemical Abstracts, Band 73, Nr. 23, 7. Dezember 1970, Columbus, Ohio, USA R. BOGNAR et al. "Heterocyclic compounds from sugars. III. Substituted thiazolidines by reaction of L-cysteine with monosaccharides" Seite 402, Spalte 1, Abstract Nr. 120832b**

(73) Patentinhaber : **Degussa Aktiengesellschaft**
**Weissfrauenstrasse 9**
**D-6000 Frankfurt am Main 1 (DE)**

(72) Erfinder : **Bethge, Horst**
**Schwalbenstrasse 2A**
**D-6450 Hanau 1 (DE)**
Erfinder : **Drauz, Karlheinz, Dr. Dipl.-Chem.**
**Flurstrasse 5**
**D-6463 Freigericht 1 (DE)**
Erfinder : **Kleemann, Axel, Dr. Dipl.-Chem.**
**Greifenhagenstrasse**
**D-6450 Hanau 9 (DE)**
Erfinder : **Martens, Jürgen, Dr. Dipl.-Chem.**
**Hochstrasse 10**
**D-8755 Alzenau (DE)**
Erfinder : **Weigel, Horst**
**Odenwaldstrasse 56**
**D-6458 Rodenbach (DE)**

**EP 0 086 258 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Trennung des Racemats (R,S)-Cystein.

(R)-Cystein, aber auch dessen Antipode (S)-Cystein besitzen Bedeutung als Zwischenprodukte für die Herstellung von Pharmazeutika oder Kosmetika und in der Peptidchemie.

Da seit kurzem (R,S)-Cystein in Form des Cystein-Hydrochlorid-Monohydrats leicht in technischem Maßstab zugänglich ist [vgl. Angew. Chem. *93,* 680 (1981)], besteht nunmehr ein besonderes Bedürfnis für ein einfaches, kostengünstiges und leistungsfähiges Verfahren zur Trennung des Racemats (R,S)-Cystein.

Das erfindungsgemäße Verfahren ist nun dadurch gekennzeichnet, daß man das (R,S)-Cystein in Wasser, einem mit Wasser mischbaren Alkohol oder Ether mit bis zu 5 Kohlenstoffatomen oder einem Gemisch solcher Lösungsmittel mit einem enantiomerenreinen Monosaccharid der allgemeinen Formel

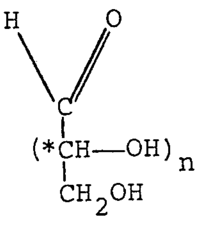

(I)

in welcher n eine ganze Zahl von 2 bis 5 und *C ein asymmetrisches Kohlenstoffatom bedeuten, wobei die absolute Konfiguration der asymmetrischen Kohlenstoffatome gleich oder verschieden ist, zu einer in 2-Stellung substituierten Thiazolidin-4-carbonsäure der allgemeinen Formel

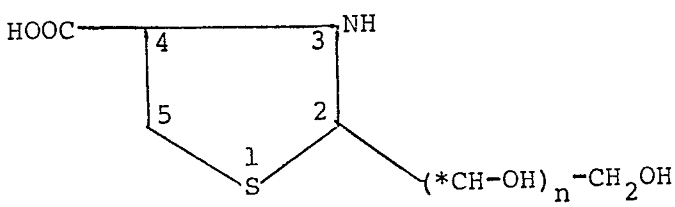

(II)

in der n und *C wieder die bereits angegebene Bedeutung haben, kondensiert, das erhaltene Diastereomerengemisch voneinander trennt, die isolierten einheitlichen Diastereomeren in Wasser, einem mit Wasser mischbaren Alkohol oder Ether mit bis zu 5 Kohlenstoffatomen oder einem Gemisch solcher Lösungsmittel zur Ringspaltung mit einer Verbindung umsetzt, welche die Gruppe $—NH_2$ enthält und zur Kondensation mit Carbonylgruppen befähigt ist, und das jeweils erhaltene enantiomerenreine Cystein als solches oder als das entsprechende Cystin isoliert.

Als enantiomerenreine Monosaccharide der allgemeinen Formel (I) für die Kondensation mit dem (R,S)-Cystein eignen sich beispielsweise bekannte Aldosen, wie L-Arabinose, D-Arabinose, L-Galaktose, D-Galaktose, L-Glucose, D-Glucose, L-Mannose, D-Mannose, L-Xylose oder D-Xylose.

Die Kondensation wird in Wasser oder in einem mit Wasser mischbaren Alkohol oder Ether mit bis zu 5 Kohlenstoffatomen, beispielsweise Methanol, Ethanol, Propan-1-ol, Propan-2-ol, Butan-1-ol, Butan-2-ol, Isobutanol, tert. Butanol, Pentan-1-ol, Tetrahydrofuran oder 1,4-Dioxan, oder in einem Gemisch solcher Lösungsmittel vorgenommen. Bevorzugte Lösungsmittel sind Wasser, Methanol oder Ethanol.

Das enantiomerenreine Monosaccharid der allgemeinen Formel (I) wird zweckmäßigerweise in einer Menge zwischen 1 und 2,5 Mol pro Mol eingesetztem (R,S)-Cystein eingesetzt. Besonders vorteilhaft ist es jedoch, das Monosaccharid und das (R,S)-Cystein in äquimolaren Mengen einzusetzen. Die Reihenfolge der Zugabe ist unkritisch.

Die Kondensation erfolgt zweckmäßigerweise bei einer Temperatur zwischen 0 °C und der Rückflußtemperatur des Reaktionsgemisches, vorzugsweise bei einer Temperatur zwischen 20 und 70 °C.

Wird nicht das freie (R,S)-Cystein, sondern ein Säureadditionssalz desselben, z. B. das Hydrochlorid, eingesetzt, so ist es erforderlich, vor der Kondensation die enthaltene Säure durch Zugabe von 1 bis 1,5 Äquivalenten einer Base, insbesondere eines tertiären Amins, wie Triethylamin oder Pyridin, zu binden.

Bei der Kondensation bildet sich die entsprechende in 2-Stellung substituierte Thiazolidin-4-carbonsäure der allgemeinen Formel (II) in Form eines Gemisches von zwei Stereoisomeren, die zueinander diastereomer sind und sich durch die Konfiguration in 4-Stellung des Thiazolidinringes unterscheiden. Die beiden Diastereomeren lassen sich in an sich bekannter Weise, z. B. durch fraktionierte Kristallisation oder durch chromatographische Methoden, voneinander trennen.

Bei der Trennung erhält man, gegebenenfalls nach ein- oder mehrfacher Umkristallisation aus einem der oben für die Kondensationsreaktion genannten Lösungsmittel oder Lösungsmittelgemische, die sterisch einheitlichen Verbindungen der allgemeinen Formel (II), von denen das eine in 4-Stellung R-Konfiguration und das andere S-Konfiguration aufweist.

Häufig kristallisiert eines der beiden Diastereomeren der allgemeinen Formel (II) nach beendeter Kondensationsreaktion bereits in der Wärme aus, im allgemeinen ist es jedoch vorteilhaft, das Reaktionsgemisch längere Zeit auf einer Temperatur zwischen 0 und 40 °C zu halten und gegebenenfalls, sofern die Kondensation in Wasser vorgenommen wurde, eines der oben genannten Lösungsmittel zuzusetzen.

Das ausgefallene Kristallisat wird durch Filtration oder Zentrifugation von der Mutterlauge abgetrennt. Es besteht im allgemeinen vollständig oder doch weit überwiegend aus einem der beiden Diastereomeren der allgemeinen Formel (II). Erforderlichenfalls kann es durch Umkristallisation weiter gereinigt werden.

Die Mutterlauge enthält normalerweise noch einen Restanteil des abgetrennten Diastereomeren, aber das andere Diastereomere ist in ihr stark angereichert. Es kann durch Einengen der Mutterlauge, längeres Stehenlassen bei niedriger Temperatur, Zusatz eines organischen Lösungsmittels oder durch die Kombination von mehreren dieser Maßnahmen zur Kristallisation gebracht werden. Es liegt im allgemeinen dann in geringerer Reinheit als das zuerst abgetrennte Diastereomere vor. Eine zusätzliche Reinigung durch Umkristallisation dürfte daher in vielen Fällen zweckmäßig sein.

Alternativ können die beiden bei der Kondensationsreaktion als Gemisch erhaltenen Diastereomeren der allgemeinen Formel (II) durch übliche präparative chromatographische Methoden getrennt werden. Dabei erfolgt eine sehr weitgehende Trennung und jedes der beiden Diastereomeren kann in hoher Reinheit isoliert werden.

Die isolierten sterisch einheitlichen Verbindungen der allgemeinen Formel (II) werden nun getrennt in einem der oben für die Kondensationsreaktion genannten Lösungsmittel oder Lösungsmittelgemische zur Spaltung des Thiazolidinringes mit einer Verbindung umgesetzt, welche eine zur Kondensation mit der Carbonylgruppe der ursprünglich eingesetzten Aldose befähigte Aminogruppe enthält.

Für die Ringspaltung geeignete Verbindungen sind demnach die typischen Carbonylreagentien, wie Hydroxylamin, z. B. in Form des Hydrochlorids, Hydrazin, z. B. in Form des Hydrats, Phenylhydrazin oder Anilin. Sie werden in einer der zur Ringspaltung eingesetzten Verbindung der allgemeinen Formel (II) mindestens äquivalenten Menge eingesetzt, können jedoch auch im Überschuß angewandt werden.

Die Ringspaltung erfolgt zweckmäßig bei einer Temperatur zwischen 50 °C und der Rückflußtemperatur des Reaktionsgemisches. Besonders rasch und vollständig verläuft sie, wenn das Reaktionsgemisch etwa 0,5 bis etwa 5 Stunden lang unter Rückfluß erhitzt wird.

Bei der Ringspaltung erhält man aus dem Diastereomeren der allgemeinen Formel (II) mit R-Konfiguration in 4-Stellung des Thiazolidinringes enantiomerenreines (R)-Cystein, aus dem Diastereomeren mit S-Konfiguration in 4-Stellung des Thiazolidinringes entsprechend enantiomerenreines (S)-Cystein.

Zur Trennung des bei der Ringspaltung gebildeten enantiomerenreinen Cysteins von dem gleichzeitig gebildeten Kondensationsprodukt aus der eingesetzten Aldose und dem zur Ringspaltung verwendeten Carbonylreagenz wird, sofern dieses Kondensationsprodukt schwerer löslich ist als das Cystein, zunächst das Kondensationsprodukt abfiltriert. Dann wird aus dem Filtrat das Cystein durch Einengen und/oder durch Einstellen des pH auf einen Wert in der Nähe des isoelektrischen Punktes in kristalliner Form isoliert.

Ist dagegen das Kondensationsprodukt aus der eingesetzten Aldose und dem zur Ringspaltung verwendeten Carbonylreagenz leichter löslich als das Cystein, so wird dieses durch Einstellen des pH auf einen Wert in der Nähe des isoelektrischen Punktes ausgefällt und abgetrennt.

Wegen der im Vergleich zu der des Cysteins geringeren Löslichkeit des Cystins kann es in manchen Fällen vorteilhafter sein, das bei der Ringspaltung gebildete enantiomerenreine (R)-Cystein bzw. (S)-Cystein nicht als solches zu isolieren, sondern es mit einem geeigneten Oxidationsmittel, beispielsweise Wasserstoffperoxid, zum (R,R)-Cystin, bzw. (S,S)-Cystin zu oxydieren und dieses zu isolieren. Das enantiomerenreine Cystin läßt sich dann bei Bedarf leicht wieder in an sich bekannter Weise zum entsprechenden Cystein reduzieren.

Ebensogut ist es aber auch möglich, nach beendeter Ringspaltung zunächst die Hauptmenge des gebildeten enantiomerenreinen Cysteins zu gewinnen und anschließend den in der Mutterlauge verbleibenden Restanteil zum entsprechenden Cystin zu oxydieren und als solches zu isolieren.

Die Erfindung soll durch die nachfolgenden Beispiele näher erläutert werden. Sofern nicht anders angegeben, bedeuten alle Prozentangaben Gewichtsprozente.

## Beispiel 1

87,5 g (0,5 Mol) (R,S)-Cystein × HCl × $H_2O$ und 90 g (0,5 Mol) D-(+)-Galaktose werden in 3,75 l Methylalkohol, 50 ml Pyridin und 50 ml Wasser 20 Minuten unter Rückfluß gekocht. Innerhalb 2,25 Stunden wird die Reaktionslösung langsam bis auf 40 °C abgekühlt und die ausgeschiedenen Kristalle werden abgesaugt. Nach Waschen mit Methylalkohol erhält man 40,5 g farblose Kristalle von (4S)-2-(D-Galakto-1,2,3,4,5-pentahydroxypentyl)-thiazolidin-4-carbonsäure, entsprechend einer Ausbeute von 57,2 % der Theorie.

$[\alpha]_D^{20} = +89,3°$ (c = 0,5, $H_2O$).

Durch Umkristallisieren aus wässrigem Ethanol steigt der Drehwert auf + 91,3°.

Der Drehwert einer authentischen, aus enantiomerenreinem (S)-Cystein und D-(+)-Galaktose hergestellten Vergleichsprobe beträgt ebenfalls + 91,3°.

Schmelzpunkt : 178 °C unter Zersetzung.

Elementaranalyse : $C_9H_{17}NO_7S$
Gefunden : % C 39,25 % H 5,93 % N 4,79
Berechnet : % C 39,14 % H 6,04 % N 4,94

Aus dem Filtrat scheidet sich bei 3-tägigem Stehen ein voluminöser Niederschlag ab. Durch Absaugen, Waschen mit Methylalkohol und Trocknen erhält man 61,5 g farblose Kristalle, in welchen die (4R)-2-(D-Galakto-1,2,3,4,5-pentahydroxypentyl)-thiazolidin-4-carbonsäure stark angereichert ist. Durch Extraktion mit warmen Methylalkohol kann sie in reiner Form isoliert werden.

$[\alpha]_D^{20}$ = − 71,2° (c = 0,5, Wasser). Schmelzpunkt : 147 bis 149 °C.

Durch Umkristallisation aus wässrigem Ethanol steigt der Drehwert auf − 78,2°.

Der Drehwert einer authentischen, aus enantiomerenreinem (R)-Cystein und (D)-(+)-Galaktose hergestellten Vergleichsprobe beträgt ebenfalls − 78,2°.

40,5 g (0,145 Mol) (4S)-2-(D-Galakto-1,2,3,4,5-pentahydroxypentyl)-thiazolidin-4-carbonsäure und 13,3 g (0,185 Mol) Hydroxylamin-hydrochlorid werden mit 700 ml Methylalkohol verrührt und unter Rückfluß zum Sieden erhitzt. Nach 20 Minuten entsteht eine klare Lösung. Nach 2 Stunden ist die Reaktion beendet. Die Reaktionslösung wird abgekühlt und durch Filtration von dem Oxim befreit. Mit Triethylamin wird das Filtrat auf pH 6 gestellt, wobei 12,7 g (S)-Cystein auskristallisieren (Ausbeute : 72,7 % der Theorie).

$[\alpha]_D^{20}$ = − 9,5° (c = 8, 1N HCl).

40,5 g (0,145 Mol) (4R)-2-(D-Galakto-1,2,3,4,5-pentahydroxypentyl)-thiazolidin-4-carbonsäure und 13,3 g (0,185 Mol) Hydroxylamin-hydrochlorid werden mit 600 ml Methanol verrührt und unter Rückfluß zum Sieden erhitzt. Nach 20 Minuten entsteht eine klare Lösung. Nach 3 Stunden ist die Reaktion beendet. Die Reaktionslösung wird abgekühlt und durch Filtration vom Oxim befreit.

Mit Triethylamin wird das Filtrat auf pH 5,5 gestellt, wobei 12,7 g (R)-Cystein auskristallisieren (Ausbeute : 72,7 % der Theorie).

$[\alpha]_D^{20}$ = + 9,8° (c = 8, 1N HCl).

## Beispiel 2

Alternativ werden 7,1 g (0,025 Mol) der in Beispiel 1 erhaltenen (4S)-2-(D-Galakto-1,2,3,4,5-pentahydroxypentyl)-thiazolidin-4-carbonsäure mit 20 ml Wasser angeteigt und mit 5,2 g einer 24 %igen wässrigen Hydrazinhydratlösung verrührt. Die Mischung wird 10 Minuten lang auf 60 °C erwärmt. Nach dem Abkühlen auf Raumtemperatur werden 1,8 g 30 %iges Wasserstoffperoxid tropfenweise zugegeben, wobei sofort (S,S)-Cystin auskristallisiert.

Nach Absaugen, Waschen mit Wasser und Trocknen erhält man 2,8 g reines (S,S)-Cystin, entsprechend einer Ausbeute von 93,3 % der Theorie.

$[\alpha]_D^{20}$ = + 218,7° (c = 1, 1N HCl).

## Beispiel 3

17,5 g (0,1 Mol) (R,S)-Cystein × HCl × $H_2O$ und 18 g (0,1 Mol) D-(+)-Mannose werden in 300 ml Methylalkohol suspendiert und nach Zugabe von 10 ml Pyridin 1 Stunde unter Rückfluß gekocht. Während des Siedens beginnt das Produkt zu kristallisieren. Nach Abkühlen bis auf 60 °C wird abgesaugt und mit Methylalkohol gewaschen.

Man erhält 9,4 g (66,4 % der Theorie) (4R)-2-(D-Manno-1,2,3,4,5-pentahydroxypentyl)-thiazolidin-4-carbonsäure als farblose Kristalle.

$[\alpha]_D^{20}$ = − 63,4° (c = 0,5, Wasser). Durch Umkristallisieren aus wässrigem Ethanol steigt der Drehwert auf − 63,8°.

Der Drehwert einer authentischen, aus enantiomerenreinem (R)-Cystein und D-(+)-Mannose hergestellten Vergleichsprobe beträgt ebenfalls − 63,8°.

Schmelzpunkt : 177 °C unter Zersetzung.

Elementaranalyse : $C_9H_{17}NO_7S$
Gefunden : % C 39,02 % H 6,08 % N 4,88
Berechnet : % C 39,14 % H 6,04 % N 4,94

Das Filtrat wird auf 20 °C abgekühlt. Dabei scheidet sich ein Gemisch der beiden Diastereomeren aus, welches durch Filtration abgetrennt und verworfen wird. Zu dem nunmehr verbliebenen Filtrat wird Essigsäureethylester zugegeben, solange sich Kristalle abscheiden. Durch Umkristallisieren aus Ethylalkohol erhält man 4,7 g (33,2 % der Theorie) an (4S)-2-(D-Manno-1,2,3,4,5-pentahydroxypentyl)-thiazolidin-4-carbonsäure in reiner Form.

$[\alpha]_D^{20}$ = + 100,5° (c = 0,5, Wasser).

Der Drehwert einer authentischen aus enantiomerenreinem (S)-Cystein und D-(+)-Mannose hergestellten Vergleichsprobe beträgt + 101,1°.

Schmelzpunkt : 153 °C unter Zersetzung.

9,4 g (0,033 Mol) (4R)-2-(D-Manno-1,2,3,4,5-pentahydroxypentyl)-thiazolidin-4-carbonsäure und 6,88 g (0,033 Mol) einer 24 %igen wässrigen Hydrazinhydratlösung werden mit 95 ml Methylalkohol 30 Minuten unter Rückfluß gekocht. Die erhaltene Suspension wird heiß filtriert und der Rückstand wird mit heißem 95 %igem Methylalkohol gewaschen. Man erhält 2,78 g (R)-Cystein (69,4 % der Theorie).

$[\alpha]_D^{25}$ = + 9,7° (c = 8, 1N HCl).

Nach Verdünnen des Filtrats mit 70 ml Wasser und Zugabe von 1,5 ml 30 %igem Wasserstoffperoxid kristallisieren 2,2 g (R,R)-Cystin aus (26,6 % der Theorie, bezogen auf die eingesetzte (4R)-2-(D-Manno-1,2,3,4,5-pentahydroxypentyl)-thiazolidin-4-carbonsäure).

$[\alpha]_D^{25}$ = − 217 (c = 1, 1N HCl).

Elementaranalyse : $C_6H_{12}O_4N_2S_2$

| | | | | |
|---|---|---|---|---|
| Gefunden : | % C 29,98 | % H 5,07 | % N 11,82 | % S 26,63 |
| Berechnet : | % C 29,99 | % H 5,03 | % N 11,66 | % S 26,69 |

4,7 g (0,001 65 Mol) (4S)-2-(D-Manno-1,2,3,4,5-pentahydroxypentyl)-thiazolidin-4-carbonsäure und 3,44 g (0,001 65 Mol) einer 24 %igen wässrigen Hydrazinhydratlösung werden mit 50 ml Methanol 45 Minuten unter Rückfluß gekocht. Die erhaltene Suspension wird heiß filtriert und der Rückstand mit 95 %igem heißen Methanol gewaschen. Man erhält 1,5 g (S)-Cystein, entsprechend einer Ausbeute von 74,9 % der Theorie.

$[\alpha]_D^{25}$ = − 9,7° (c = 8, 1N HCl).

## Beispiel 4

35 g (0,2 Mol) (R,S)-Cystein × HCl × $H_2O$, 39,6 g (0,2 Mol) D-(+)-Glucose × $H_2O$ und 20,2 g (0,2 Mol) Triethylamin werden in 80 ml Wasser und 20 ml Methylalkohol bei 60 °C gelöst. Die Lösung bleibt 20 Stunden bei Raumtemperatur stehen. Die gebildeten Kristalle werden abfiltriert und mit Methylalkohol gewaschen.

Man erhält 5,1 g (36,0 % der Theorie) (4R)-2-(D-Gluco-1,2,3,4,5-pentahydroxypentyl)-thiazolidin-4-carbonsäure.

$[\alpha]_D^{20}$ = − 84,6° (c = 0,5, Wasser). Durch Umkristallisieren aus wässrigem Ethanol steigt der Drehwert auf − 89,6°.

Der Drehwert einer authentischen, aus enantiomerenreinem (R)-Cystein und D-(+)-Glucose hergestellten Vergleichsprobe beträgt ebenfalls − 89,6°.

Schmelzpunkt : 162 °C unter Zersetzung.

Elementaranalyse : $C_9H_{17}NO_7S$

| | | | |
|---|---|---|---|
| Gefunden : | % C 39,20 | % H 6,02 | % N 4,83 |
| Berechnet : | % C 39,14 | % H 6,04 | % N 4,94 |

Man engt das beim Abtrennen der (4R)-2-(D-Gluco-1,2,3,4,5-pentahydroxypentyl)-thiazolidin-4-carbonsäure angefallene Filtrat unter vermindertem Druck auf 90 ml ein. Nach Stehen über Nacht bei 15 °C wird das auskristallisierende Gemisch aus (4R)- und (4S)-2-(D-Gluco-1,2,3,4,5-pentahydroxypentyl)-thiazolidin-4-carbonsäure abgenutscht. Das dabei anfallende Filtrat wird am Rotationsverdampfer bei 25 mbar und Erwärmung mit einem Wasserbad zur Trockne eingeengt. Der Rückstand wird aus Wasser umkristallisiert. Man erhält reine (4S)-2-(D-Gluco-1,2,3,4,5-pentahydroxypentyl)-thiazolidin-4-carbonsäure in Form farbloser Kristalle. Ausbeute : 4,8 g (34 % der Theorie).

Schmelzpunkt : 168 °C unter Zersetzung.

$[\alpha]_D^{20}$ = + 85,1° (c = 0,5, Wasser).

Der Drehwert einer authentischen, aus enantiomerenreinem (S)-Cystein und D-(+)-Glucose hergestellten Vergleichsprobe beträgt + 85,2°.

5,1 g (0,018 Mol) (4R)-2-(D-Gluco-1,2,3,4,5-pentahydroxypentyl)-thiazolidin-4-carbonsäure und 1,87 g (0,027 Mol) Hydroxylamin-hydrochlorid werden in einer Mischung aus 10 ml Wasser und 40 ml Methylalkohol 3,5 Stunden unter Rückfluß zum Sieden erhitzt. Nach dem Erkalten wird eine leichte Trübung durch Filtration entfernt und das Filtrat wird mit Triethylamin auf pH 6 gestellt, wobei 1,62 g (R)-Cystein auskristallisieren, (entsprechend einer Ausbeute von 74,3 % der Theorie).

$[\alpha]_D^{25}$ = + 9,8° (c = 8, 1N HCl).

4,8 g (0,017 Mol) (4S)-2-(D-Gluco-1,2,3,4,5-pentahydroxypentyl)-thiazolidin-4-carbonsäure und 1,87 g (0,027 Mol) Hydroxylamin-hydrochlorid werden in einer Mischung aus 10 ml Wasser und 40 ml Methanol 4 Stunden unter Rückfluß gekocht. Nach dem Abkühlen auf 20 °C wird eine Trübung durch Filtration entfernt und das Filtrat mit Triethylamin auf pH 5,5 gestellt, wobei 1,6 g (79 % der Theorie) (S)-Cystein auskristallisieren.

$[\alpha]_D^{25}$ = − 9,7° (c = 8, 1N HCl).

Beispiel 5

1,2 g (0,01 Mol) (R,S)-Cystein und 1,5 g (0,01 Mol) L-(+)-Arabinose werden in 5 ml Wasser 30 Minuten auf 80 °C erwärmt. Die erhaltene Reaktionslösung wird dünnschichtchromatographisch [Kieselgel 60 (E. Merck, Darmstadt), 2 mm Schichtdicke] getrennt, wobei als Laufmittel ein Gemisch aus 5 Volumenteilen n-Butanol, 2 Volumenteilen Ethanol und 4 Volumenteilen Wasser eingesetzt wird. Das Diastereomerenpaar wird so getrennt (Rf = 0,17 und 0,29).

Die Zone bei Rf = 0,17 wird von der DC-Platte abgelöst und die (4R)-2-(L-Arabino-1,2,3,4-tetrahydroxybutyl)-thiazolidin-4-carbonsäure daraus durch Extraktion mit Wasser, nachfolgendes Abfiltrieren des Kieselgels und Einengen zur Trockne erhalten. Der Rückstand wird aus wässrigem Ethanol umkristallisiert und besteht aus (4R)-2-(L-Arabino-1,2,3,4-tetrahydroxybutyl)-thiazolidin-4-carbonsäure.

Schmelzpunkt : 161 °C.

Das so erhaltene Produkt wird mit 0,52 g (0,007 5 Mol) Hydroxylamin-hydrochlorid in einer Mischung aus 5 ml Wasser und 15 ml Methanol 3,5 Stunden unter Rückfluß zum Sieden erhitzt. Nach dem Abkühlen auf 20 °C wird die Trübung durch Filtration entfernt und das Filtrat mit Triethylamin auf pH 6 gestellt, wobei 0,4 g (R)-Cystein auskristallisieren (67 % der Theorie).

$[\alpha]_D^{20} = + 9{,}3°$ (c = 12, 2N HCl)

Die Zone bei Rf = 0,29 wird von der DC-Platte abgelöst und mit Wasser extrahiert. Das Kieselgel wird abfiltriert, und das Filtrat zur Trockne eingeengt. Zur Bestimmung des Schmelzpunktes wird ein kleiner Teil des Rückstandes aus wässrigem Ethanol umkristallisiert.

Schmelzpunkt : 164 °C.

Die so erhaltene (4S)-2-(L-Arabino-1,2,3,4-tetrahydroxybutyl)-thiazolidin-4-carbonsäure wird mit 0,52 g (0,007 5 Mol) Hydroxylamin-hydrochlorid in einer Mischung aus 4 ml Wasser und 10 ml Methanol 3,5 Stunden am Rückfluß gekocht. Man läßt auf 20 °C abkühlen, filtriert durch zwei Faltenfilter und stellt den pH-Wert des Filtrats auf 6 ein. Dies geschieht durch Zugabe von Triethylamin. Es kristallisieren 0,36 g, entsprechend 60 % der Theorie (S)-Cystein aus.

$[\alpha]_D^{20} = - 9{,}4°$ (c = 12, 2N HCl).

## Ansprüche

1. Verfahren zur Trennung des Racemats (R,S)-Cystein, dadurch gekennzeichnet, daß man das (R,S)-Cystein in Wasser, einem mit Wasser mischbaren Alkohol oder Ether mit bis zu 5 Kohlenstoffatomen oder einem Gemisch solcher Lösungsmittel mit einem enantiomerenreinen Monosaccharid der allgemeinen Formel

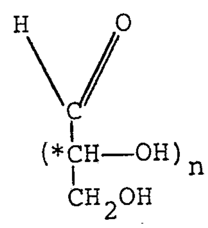

(I)

in welcher n eine ganze Zahl von 2 bis 5 und *C ein asymmetrisches Kohlenstoffatom bedeuten, wobei die absolute Konfiguration der asymmetrischen Kohlenstoffatome gleich oder verschieden ist, zu einer in 2-Stellung substituierten Thiazolidin-4-carbonsäure der allgemeinen Formel

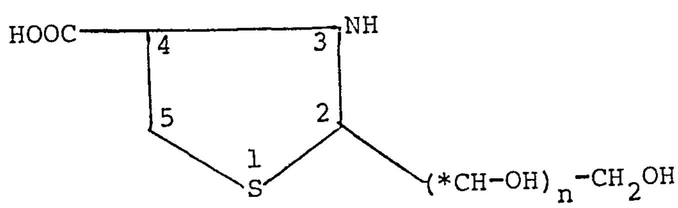

(II)

in der n und *C wieder die bereits angegebene Bedeutung haben, kondensiert, das erhaltene Diastereomerengemisch voneinander trennt, die isolierten einheitlichen Diastereomeren in Wasser, einem mit Wasser mischbaren Alkohol oder Ether mit bis zu 5 Kohlenstoffatomen oder einem Gemisch solcher Lösungsmittel zur Ringspaltung mit einer Verbindung umsetzt, welche die Gruppe $—NH_2$ enthält und zur Kondensation mit Carbonylgruppen befähigt ist, und das jeweils erhaltene enantiomerenreine Cystein als solches oder als das entsprechende Cystin isoliert.

2. Verfahren nach Anspruch 1, dadurch gekennzeichnet, daß man die Kondensation des (R,S)-Cysteins mit dem Monosaccharid der allgemeinen Formel (I) in Wasser, Methanol oder Ethanol, oder in einem Gemisch aus Wasser und Methanol oder Ethanol vornimmt.

3. Verfahren nach Anspruch 1 oder 2, dadurch gekennzeichnet, daß man die Kondensation des (R,S)-Cysteins mit dem Monosaccharid der allgemeinen Formel (I) bei einer Temperatur zwischen 20 und 70 °C vornimmt.

4. Verfahren nach einem der Ansprüche 1 bis 3, dadurch gekennzeichnet, daß man die Ringspaltung der Verbindung der allgemeinen Formel (II) in Wasser, Methanol oder Ethanol, oder in einem Gemisch aus Wasser und Methanol oder Ethanol vornimmt.

5. Verfahren nach einem der Ansprüche 1 bis 4, dadurch gekennzeichnet, daß man die Ringspaltung der Verbindung der allgemeinen Formel (II) durch Erhitzen auf die Rückflußtemperatur des Reaktionsgemisches vornimmt.

## Claims

1. A process for the separation of the racemate (R,S)-cysteine, characterised in that the (R,S)-cysteine is condensed in water, a water-miscible alcohol or ether having up to 5 carbon atoms or a mixture of solvents of this type with an enantiomer-pure monosaccharide corresponding to the general formula

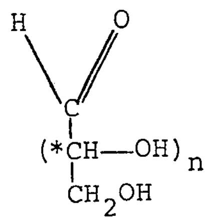

(I)

wherein n represents an integer from 2 to 5 and *C represents an asymmetric carbon atom, the absolute configuration of the asymmetric carbon atoms being the same or different, to produce a thiazolidine-4-carboxylic acid which is substituted in the 2-position and which corresponds to the general formula

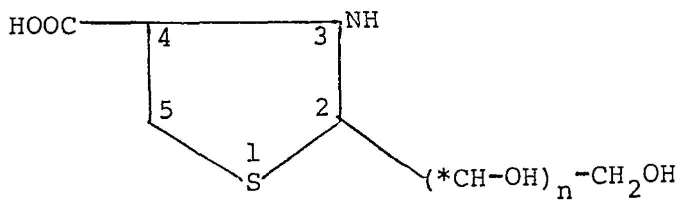

(II)

wherein n and *C are as defined above, the resulting diastereomer mixture is separated, the isolated uniform diastereomers are reacted in water, a water-miscible alcohol or ether having up to 5 carbon atoms or a mixture of solvents of this type with a compound which contains the group $—NH_2$ and is capable of condensation with carbonyl groups to produce a ring cleavage, and the enantiomer-pure cysteine which is obtained in each case is isolated as such or as the corresponding cystine.

2. A process according to claim 1, characterised in that the (R,S)-cysteine is condensed with the monosaccharide corresponding of the general formula (I) in water, methanol or ethanol, or in a mixture of water and methanol or ethanol.

3. A process according to claim 1 or 2, characterised in that the (R,S)-cysteine is condensed with the monosaccharide corresponding to the general formula (I) at a temperature of from 20 to 70 °C.

4. A process according to one of claims 1 to 3, characterised in that the ring cleavage of the compound corresponding to the general formula (II) is effected in water, methanol, or in a mixture of water and methanol or ethanol.

5. A process according to one of claims 1 to 4, characterised in that the ring cleavage of the compound corresponding to the general formula (II) is effected by heating to the reflux temperature of the reaction mixture.

## Revendications

1. Procédé de dédoublement de la (R,S)-cystéine racémique, caractérisé en ce que l'on condense la (R,S)-cystéine dans l'eau, un alcool miscible à l'eau ou un éther avec au plus 5 atomes de carbone, ou un mélange de ces solvants, avec un monosaccharide exempt d'énantiomère de formule générale

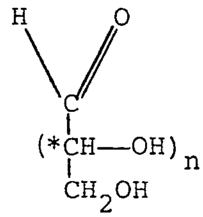

(I)

où n représente un nombre entier de 2 à 5 et *C un atome de carbone asymétrique, la configuration absolue de l'atome de carbone asymétrique étant identique ou différente, en un acide thiazolidine-4-carboxylique substitué en position 2, de formule générale

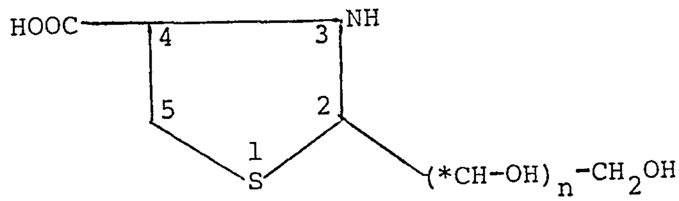

(II)

où n et *C ont la même signification que précédemment, et l'on sépare les deux diastéréoisomères obtenus en mélange, on fait réagir les diastéréoisomères purs isolés dans l'eau, un alcool miscible à l'eau ou un éther, avec au plus 5 atomes de carbone, ou un mélange de ces solvants, pour réaliser l'ouverture du cycle, avec un composé qui contient le groupe $—NH_2$ et qui est apte à la condensation avec le groupe carbonyle, et l'on isole chaque fois la cystéine exempte d'énantiomère ainsi obtenue, telle quelle ou sous forme de la cystéine correspondante.

2. Procédé selon la revendication 1, caractérisé en ce que l'on effectue la condensation de la (R,S)-cystéine avec le monosaccharide de formule générale (I) dans l'eau, le méthanol ou l'éthanol, ou dans un mélange d'eau, et de méthanol ou d'éthanol.

3. Procédé selon l'une des revendications 1 et 2, caractérisé en ce que l'on effectue la condensation de la (R,S)-cystéine avec le monosaccharide de formule générale (I) à la température comprise entre 20 et 70 °C.

4. Procédé selon l'une quelconque des revendications 1 à 3, caractérisé en ce que l'on effectue l'ouverture du cycle du composé de formule générale (II) dans l'eau, le méthanol ou l'éthanol, ou dans un mélange d'eau et de méthanol ou d'éthanol.

5. Procédé selon l'une des revendications 1 à 4, caractérisé en ce que l'on effectue l'ouverture du cycle du composé de formule générale (II) par chauffage à la température de reflux du mélange réactionnel.